# EUROPEAN PATENT SPECIFICATION

(11) **EP 2 701 802 B1**
(45) Date of publication and mention of the grant of the patent: **25.03.2015**
(21) Application number: 11716924.3
(22) Date of filing: 29.04.2011
(51) Int. Cl.: A61N 5/10

(54) **METHOD FOR CALIBRATION AND QA**
VERFAHREN FÜR KALIBRIERUNG UND QA
PROCÉDÉ DE CALIBRAGE ET ASSURANCE DE QUALITÉ

(43) Date of publication of application: 05.03.2014
(73) Proprietor: Elekta AB (PUBL), 103 93 Stockholm (SE)
(72) Inventor: CARLSSON, Per, S-183 56 Täby (SE)
(74) Representative: Groth & Co. KB
(86) International application number: PCT/EP2011/056842
(87) International publication number: WO 2012/146301

(56) References cited:
- US-A- 5 281 232
- US-A1- 2005 096 515
- FORSTER D M C ET AL: "STEREOTACTIC RADIOSURGERY", SURGERY, MEDICINE PUBLISHING, ABINGTON. / ELSEVIER IMPRINT, GB, no. 97, 1 October 1991 (1991-10-01), pages 2323-2324, XP000228581, ISSN: 0263-9319

## Description

### Field of the invention

The present invention relates to the field of radiation therapy. In particular, the invention concerns a method of calibrating a positioning system in a radiation therapy system comprising a radiation therapy unit having a fixed radiation focus point.

### Background of the invention

The development of surgical techniques has made great progress over the years. For instance, for patients requiring brain surgery, non-invasive surgery is now available which is afflicted with very little trauma to the patient.

One system for non-invasive surgery is sold under the name of Leksell Gamma Knife^{®}, which provides such surgery by means of gamma radiation. The radiation is emitted from a large number of fixed radioactive sources and is focused by means of collimators, i.e. passages or channels for obtaining a beam of limited cross section, towards a defined target or treatment volume. Each of the sources provides a dose of gamma radiation which is insufficient to damage intervening tissue. However, tissue destruction occurs where the radiation beams from all radiation sources intersect or converge, causing the radiation to reach tissue-destructive levels. The point of convergence is hereinafter referred to as the "focus point".

A patient to be treated with radiation therapy is fixated to a positioning system using a stereotactic fixation unit. Hence, the stereotactic fixation unit immobilizes a treatment volume in the patient in relation to the positioning system, i.e. immobilizes a portion of the patient containing a tissue area to be treated. For example, when the treatment area or volume is a portion of tissue within the head of a patient, the stereotactic fixation unit generally constitutes a head fixation frame which, for example, may be fixed to the skull of the patient, e.g. by fixation screws or the like. Then, the coordinates of the stereotactic fixation unit is defined by a stereotactic fixation unit coordinate system, which through the fixed relationship with the treatment volume also is used for defining the outlines of the treatment volume. In operation, the stereotactic fixation unit, and hence the stereotactic fixation unit coordinate system, is moved in relation to the fixed radiation focus point such that the focus point is accurately positioned in the intended coordinate of the fixation unit coordinate system.

Examples of such a stereotactic fixation unit and coordinate system include the Leksell stereotactic head frame and the Leksell XYZ coordinate system, respectively. The Leksell XYZ coordinate system is a Cartesian coordinate system defined by three orthogonal axes perfectly aligned with the frame of a stereotactic fixation unit, which is arranged with three orthogonal sides. In relation to a patient, the x-axis extends in the medial-lateral direction of the patient, the y-axis extends in the anterior-posterior direction, and the z-axis extends in the cranial-caudal direction.

In other words, if a patient is properly positioned in the Leksell XYZ coordinate system, the x-axis would run from ear to ear, the z-axis from head to toe, and the y-axis from back to front of the patient.

In connection with radiation therapy in radiation therapy systems, the therapy is planned in a treatment planning system. The treatment volume of the patient is scanned using an imaging system, for example, a cone beam computed tomography (CBCT) system and the scanned images are input to the treatment planning system. Computed tomography (CT) imaging, also referred to as a computed axial tomography (CAT) scan, involves the use of rotating x-ray equipment, combined with a digital computer, to obtain images of the body. Using CT imaging, cross sectional images of body organs and tissues can be produced. Using CT imaging, not only can physicians confirm that tumors exist, but they can also pinpoint their locations, accurately measure the size of tumors, and determine whether or not they've spread to neighboring tissues. In addition to the diagnosis of certain cancers, CT imaging is used for planning and administering radiation cancer treatments, as well as for planning certain types of surgeries. Using CBCT images a volumetric reconstruction of the treatment volume can be created, which can be used in planning the treatment. To this end, the volumetric reconstruction of the treatment volume must be exactly related to the focus position of the radiation therapy system and the positioning system.

However, the CBCT reconstruction is made with relation to the rotation axis of the imaging system and the rotation axis of the CBCT system and the stereotactic fixation unit coordinate system are not aligned but will have variation due to, for example, manufacturing tolerances. Such angular variations between the CBCT coordinate system and the stereotactic fixation unit coordinate system can, for example, lead to positioning errors when the patient is fixated to the positioning system and positioned within the radiation unit for a therapy session.

Thus, there is need of a method and system for determining and compensating for deviations between a coordinate system of an imaging system, such as a CBCT system, and the stereotactic fixation unit coordinate system.

### Summary of the invention

An object of the present invention is to provide a system and method for compensating for deviations between a coordinate system of an imaging system, such as a CBCT system, and a stereotactic fixation unit coordinate system.

This and other objects are achieved by providing a calibration method having the features defined in the independent claim. Preferred embodiments are defined in the dependent claims.

According to an aspect of the present invention, there is provided a method for calibrating an imaging system for capturing images of a patient in connection with treatment planning or treatment in a radiation therapy system, which radiation therapy system comprises a radiation therapy unit having a fixed radiation focus point. Further, a positioning system for positioning a treatment volume in a patient in relation to the fixed focus point in the radiation therapy unit along three substantially orthogonal motional axes is also included in the radiation therapy system, wherein the positioning system includes a fixation arrangement for releasably and firmly engaging a stereotactic fixation unit for immobilizing a treatment volume in a patient in relation to the positioning system. The method comprises the steps of:
- releasably attaching a calibration tool to the fixation arrangement, wherein the calibration tool is positioned in a stereotactic fixation unit coordinate system related to the positioning system;
- performing an image scanning procedure with the imaging system, wherein a set of images are captured of an area including the calibration tool;
- determining a position of the calibration tool in a coordinate system related to the imaging system using the set of images; and
- calculating a position difference between the determined position of the calibration tool in the coordinate system related to the imaging system and a position of the calibration tool in the stereotactic fixation unit coordinate system, thereby determining a relationship between the coordinate system related to the imaging system and the position of the calibration tool in the stereotactic fixation unit coordinate system.

According to a second aspect of the present invention, there is provided a system for calibrating an imaging system for capturing images of a patient in connection with treatment planning or treatment in a radiation therapy system, which radiation therapy system comprises a radiation therapy unit having a fixed radiation focus point, and a positioning system for positioning a treatment volume in a patient in relation to the fixed focus point in the radiation therapy unit along three substantially orthogonal motional axes, wherein the positioning system includes fixation arrangements for releasably and firmly engaging a stereotactic fixation unit for immobilizing a treatment volume in a patient in relation to the positioning system. The system for calibration comprises a calibration tool arranged to be releasably attached to the fixation arrangement, wherein the calibration tool is positioned in a stereotactic fixation unit coordinate system related to the positioning system and a processing unit. The processing unit is configured to obtain a set of images of an area including the calibration tool captured by the imaging device from the imaging system, to determine a position of the calibration tool in a coordinate system related to the imaging system using the set of images; and to calculate a position difference between the determined position of the calibration tool in the coordinate system related to the imaging system and a position of the calibration tool in the stereotactic fixation unit coordinate system to determine a relationship between the coordinate system related to the imaging system and the position of the calibration tool in the stereotactic fixation unit coordinate system.

The present invention is based on the insight that there are angular variations or deviations between a coordinate system of an imaging system, such as a CBCT system, and the stereotactic coordinate system that defines the treatment positions due to, for example, manufacturing tolerances. The CBCT system is used to capture images of the patient and the treatment volume and the reconstructed image of the treatment volume must therefore relate to the focus position of the therapy unit and the patient positioning system position. The CBCT coordinates are physically offset from the focus and it is not possible to mechanically know from tolerances what position the CBCT system has in relation to the focus position. Thus, these variations or deviations cause positioning errors when the patient is translated into the radiation therapy unit for a treatment. Even a very small sinusoidal error may result in large deviations if the patient is translated a large distance and may hence cause large positioning errors. These insights have lead to the invention and the idea of determining and compensating for the deviations between a coordinate system of the imaging system, such as a CBCT system, and the stereotactic coordinate system that defined the treatment positions. Using the determined deviation, the position and rotation of the reconstructed object can be determined in relation to the stereotactic coordinate system. In order to determine the deviation, a calibration tool that easily can be aligned and positioned exactly in the stereotactic coordinate system and thereby securely be kept still during image acquisition is used. Preferably, the calibration tool is mounted or attached to a stereotactic fixation unit of the patient positioning system. The stereotactic fixation unit for immobilizing a treatment volume is in fixed engagement with the patient positioning system and cannot be translated or rotated in relation to the positioning system. The position of the calibration tool in a coordinate system related to the imaging system is determined using a set of images captured by means of the imaging system.

The position determination of the calibration tool is made more accurate and fast by observing or identifying a recognizable part of the calibration tool having a defined position relative the patient position system or at least one reference object or mark of the calibration tool having defined positions relative the patient position system.

In preferred embodiments of the present invention, a calibration tool having at least three ball bars attachable to the fixation unit is used. Each ball bar has a known position (coordinates) in the stereotactic coordinate system. By identifying these reference objects (i.e. ball bars) in the reconstructed volume, the position of the calibration tool can be determined with respect to the imaging system coordinate system. Thereafter, a position difference between the determined position of the calibration tool in the imaging system coordinate system and the known position of the calibration tool in the stereotactic fixation unit coordinate system can be calculated. Thereby, a relationship between the imaging system coordinate system and the known position of the calibration tool in the stereotactic fixation unit coordinate system can be determined.

According to an embodiment of the present invention, the calibration tool comprises: attachment means for enabling releasable attachment to the fixation arrangement of the patient positioning system; and reference objects having a shape enabling a position determination in six dimensions. In embodiments of the present invention, the calibration tool comprises at least three reference objects each including a rod attached to a base plate comprising the attachment means and a ball attached to respective rod.

According to embodiments of the present invention, a calibration of the imaging system is performed including determining a rotational axis of the imaging system. This calibration step may be performed before a session to determined the deviation between the imaging system coordinate system and the stereotactic coordinate system id performed.

According to an embodiment of the present invention, a position correction based on the calculated position difference is determined. The position correction corresponds to a movement correction for the imaging system relative the positioning system required to transform a position in the coordinate system of the imaging system to the corresponding position in the stereotactic fixation unit coordinate system.

According to embodiment of the present invention, the determining of a position correction comprises determining the movement correction as three rotations with respect to the respective axes of the stereotactic fixation unit coordinate system and three translations along the respective axes of the stereotactic coordinate system.

According to embodiments of the present invention, the determining of a position of the calibration tool in the imaging system coordinate system includes identifying predetermined reference objects of the calibration tool in the volumetric reconstruction, wherein the reference objects have known coordinates in the stereotactic fixation unit coordinate system; an determining the coordinates for each identified reference object in the volumetric reconstruction with respect to the coordinate system of the imaging system.

According to embodiments of the present invention, the calculation of a position difference comprises comparing coordinates for each identified reference object in the coordinate system of the imaging system and the corresponding coordinates of the reference objects in the stereotactic fixation unit coordinate system; and calculating the position difference between the determined position of the calibration tool in the coordinate system related to the imaging system and a position of the calibration tool in the stereotactic fixation unit coordinate system based on the comparison.

As readily understood by the person skilled in the art, various known methods for determining the radiation focus point could be used, of which some have been described above. However, the present invention is not restricted to the particular examples shown and described herein, but any suitable measurement method for determining the radiation focus point is contemplated within the scope of the present invention.

### Brief description of the drawings

Preferred embodiments of the invention will now be described in greater detail with reference to the accompanying drawings, in which
Fig. 1 schematically illustrates the general principle of a radiation therapy system suitable for calibration using the present invention;
Fig. 2 schematically illustrates an embodiment of the system according to present invention implemented in the radiation therapy system of Fig. 1;
Fig. 3 schematically illustrates an embodiment of the calibration tool according to the present invention;
Fig. 4 is a flow chart illustrating the overall steps of the method according to the present invention;
Fig. 5 is a flow chart illustrating the steps of an embodiment of the method according to the present invention;
Fig. 6 schematically illustrates another embodiment of the calibration tool according to the present invention;
Fig. 7 schematically illustrates a further embodiment of the calibration tool according to the present invention; and
Fig. 8 schematically illustrates yet another embodiment of the calibration tool according to the present invention;

### Description of preferred embodiments

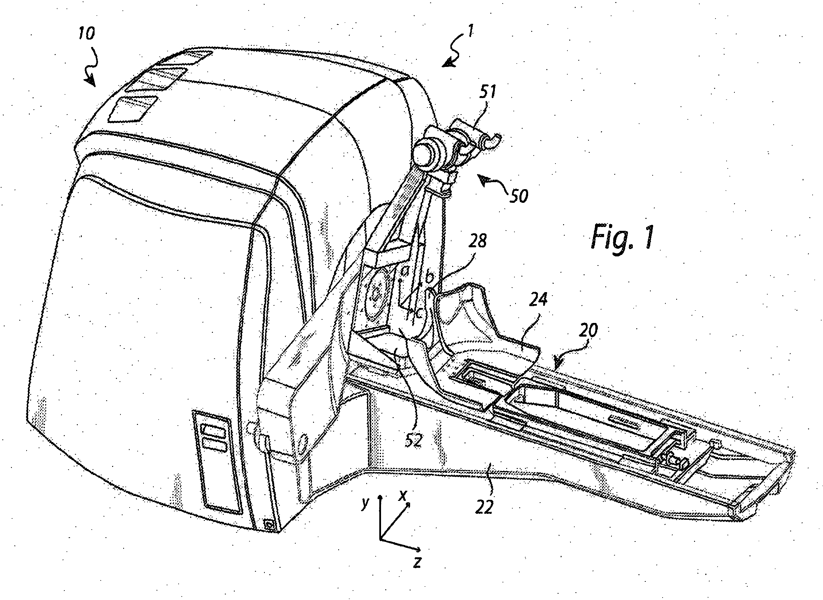

With reference to Fig. 1, a radiation therapy system 1 for which the present invention is applicable comprises a radiation unit 10 and a patient positioning unit 20. In the radiation unit 10, there are provided radioactive sources, radioactive source holders, a collimator body, and external shielding elements. The collimator body comprises a large number of collimator channels directed towards a common focus point, in a manner as is commonly known in the art.

The collimator body also acts as a radiation shield preventing radiation from reaching the patient other than through the collimator channels. Examples of collimator arrangements in radiation therapy systems applicable to the present invention can be found in WO 2004/06269 A1. However, the present invention is also applicable to radiation therapy systems using other arrangements for collimating radiation into a fixed focus point, such as is disclosed in US Patent No. 4,780,898.

The patient positioning unit 20 comprises a rigid framework 22, a slidable or movable carriage 24, and motors (not shown) for moving the carriage 24 in relation to the framework 22. The carriage 24 is further provided with a patient bed (not shown) for carrying and moving the entire patient. At one end of the carriage 24, there is provided a fixation arrangement 28 for receiving and fixing a stereotactic fixation unit (not show), either directly or via an adapter unit (not shown), and thereby preventing the stereotactic fixation unit from translational or rotational movement in relation to the movable carriage 24. The patient can be translated using the patient positioning unit 20 in the coordinate system of the radiation therapy system 1 or the patient positioning unit 20, i.e. along the three orthogonal axes x, y, and z shown in Fig. 1.

An imaging system 50 for capturing images of a patient, for example, in connection with treatment planning or treatment is arranged or located at the radiation unit 10, for example, a cone beam computed tomography (CBCT) system. The imaging system 50 includes an X-ray source 51 and a detector 52. The X-ray source 51 and detector 52 are arranged to rotate around a rotation axis c (see Fig. 1) of a coordinate system (a, b, c) of the imaging system 50 to capture images of a patient located on the patient bed 26 at different angles. Ideally, the X-ray source 51 and the detector 52 rotate around the z-axis of the patient positioning unit 20, which is aligned with the rotation axis c of the imaging system 50. However, in practice, there are, for example, alignments errors due manufacturing tolerances leading to a misalignment between the coordinate system of the patient positioning unit 20 and the imaging system 50 and accordingly the c-axis is not aligned with the z-axis.

In computed tomography, a three-dimensional image is generated by rotating the imaging system around the object in very small steps (e.g. <1°) around a single axis of rotation while taking a series of two-dimensional X-ray images. In other applications, the object is rotated around the imaging in small steps. Normally, the imaging device or the object is rotated, for example, 180° or 360° around the object or imaging device, respectively. Afterwards, a final three-dimensional image can be numerically reconstructed based on the two- dimensional images and can be displayed either as a series of sectional images or a three-dimensional image.

As can be understood from Fig. 1, the described embodiment concerns a radiation therapy system for providing gamma radiation therapy to a target volume in the head of human patient. Such therapy is often referred to as stereotactic surgery. During therapy, the patient head is fixed in a stereotactic fixation unit, for example, using a bite-block and a fixation unit in the form of a stereotactic head frame, which comprises engagement points adapted for engagement with the fixation arrangement 28 of the radiation therapy system. Thus, during the stereotactic surgery, the head of the patient is fixed in the stereotactic frame, which in turn is fixedly attached to the patient positioning system via the fixation arrangement 28. During movement of the treatment volume in the head of the patient in relation to the radiation focus point, along the three orthogonal axes x, y, and z shown in Fig. 1, the entire patient is moved along the axes. Thus, there is no relative movement between the head frame and the carriage 24 of the patient positioning system 20.

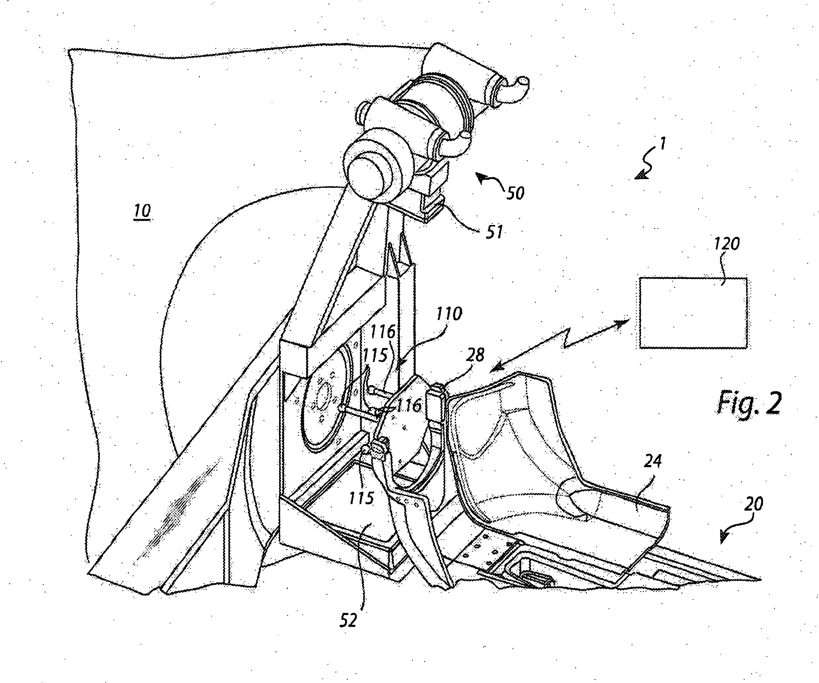

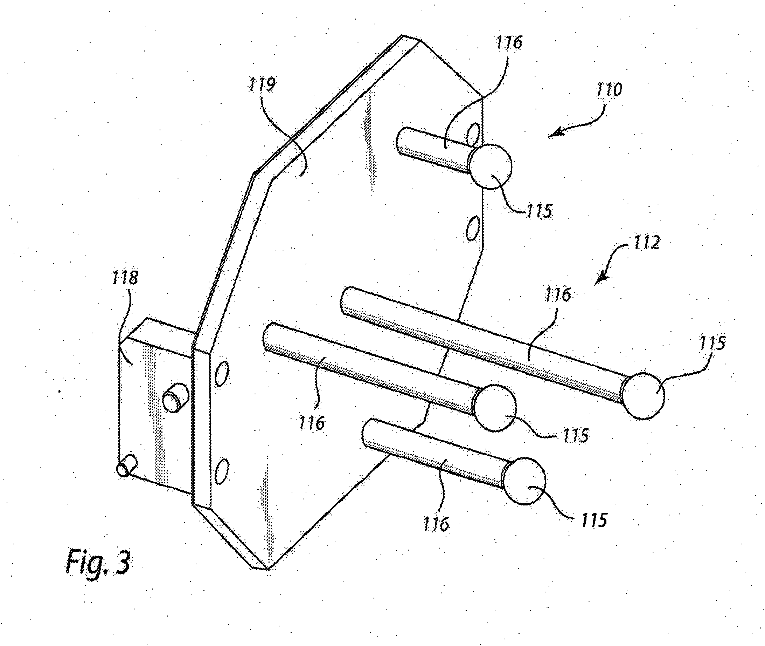

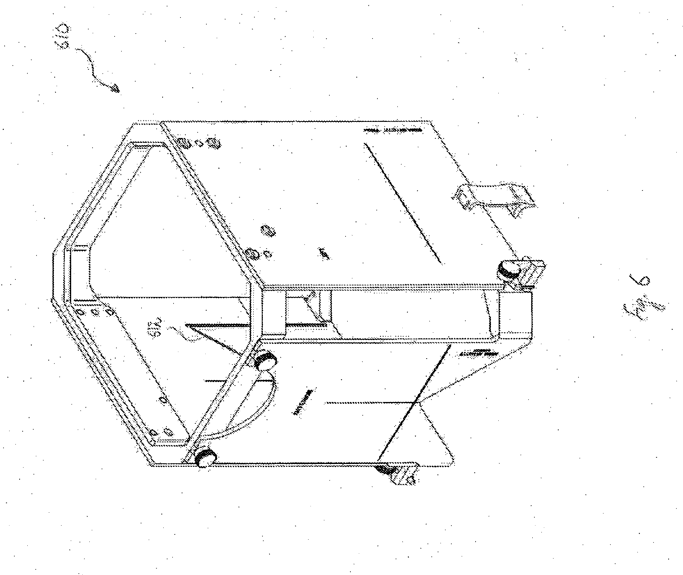

Turning now to Fig. 2, an embodiment of the system according to the present invention will be discussed. In Fig. 2, the system 100 according to the present invention is schematically shown together with a schematically illustrated radiation unit 10 and an imaging system 50. The system 1 according to the present invention comprises on the general level a calibration tool 110 arranged to be releasably and firmly attached to the fixation arrangement 28 of the radiation therapy system and processing unit 120, for example, a personal computer (PC). In Fig. 3, a more detailed view of an embodiment of the calibration tool 110 is shown. Further embodiments of the calibration tool are shown in Fig. 6 - 8.

The calibration tool 110 is arranged to be easily aligned and positioned exactly in the stereotactic fixation unit coordinate system. By attaching the calibration tool 110 firmly by means of attachment means 118 without any possibility to movement of the calibration tool 110 relative to the patient positioning unit 20, it can be secured that the calibration tool is located at a defined and predetermined position, x_{cal.tool}, y_{cal.tool}, and z_{cal.tool}, in the stereotactic fixation unit coordinate system and that it is kept still during image acquisition.

Preferably, the calibration tool 110 comprises at least one reference object or mark 112 having predetermined or known positions, respectively, in the stereotactic fixation unit coordinate system when the tool 110 is attached to the fixation arrangement 28. That is, the reference objects or marks 112 have predetermined coordinates in the stereotactic fixation unit coordinate system. The reference marks or objects 112 is arranged or shaped such that they can be identified in images captured by the imaging system 50 and/or the reconstructed volume (i.e. the reconstructed volume created by the captured images). Furthermore, the reference objects or marks 112 are arranged or shaped such that they can be identified in images captured by the imaging system 50 and/or the reconstructed volume and positioned in the images or volume in six dimensions, i.e. translation along the three axes x, y, and z and rotation around the axes.

In the embodiment of the calibration tool 110 shown in Fig. 2 and 3, the calibration tool 110 comprises four reference objects 112 each including a rod 116 provided with a ball 115 attached on a plate 119. Each reference object 112 has a predetermined position in the stereotactic fixation unit coordinate system when the calibration tool 110 is attached to the fixation arrangement 28.

In Figs. 6 - 8, respectively, further embodiments of the calibration tool according to the present invention are shown.

In Fig. 6, a calibration tool 610 comprising one reference object 612 is shown. In this embodiment, the reference object 612 is an N-shaped slit or marking, which, however, may have other shapes. The reference object 612 has a predetermined position in the calibration tool 612 and thus has a predetermined position in the stereotactic fixation unit coordinate system when the calibration tool 610 is attached to the fixation arrangement 28.

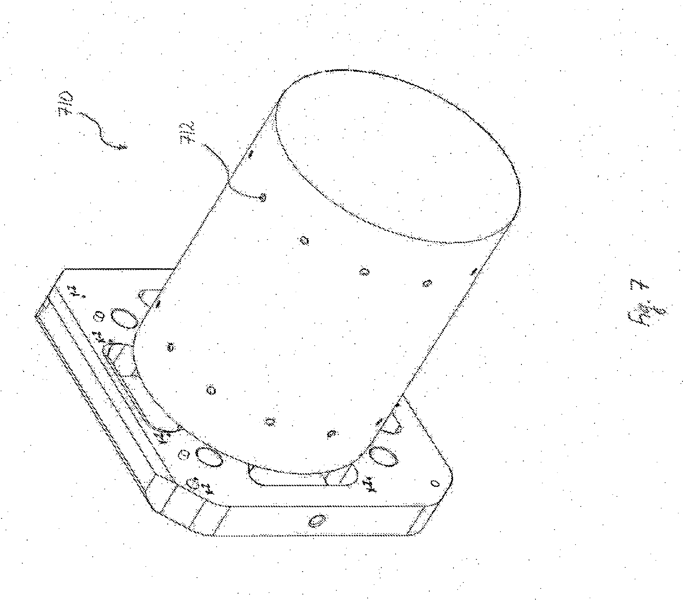

In Fig. 7, a calibration tool 710 comprising reference objects 712 is shown. In this embodiment, the reference objects 712 are, for example, balls, cylinder shaped elements or marks provided on the inner or outer surface of the cylinder shaped calibration tool 710 or balls provided inside the cylinder shaped calibration tool 710. The reference objects 712 have predetermined positions in the calibration tool 712 and thus have predetermined positions in the stereotactic fixation unit coordinate system when the calibration tool 710 is attached to the fixation arrangement 28.

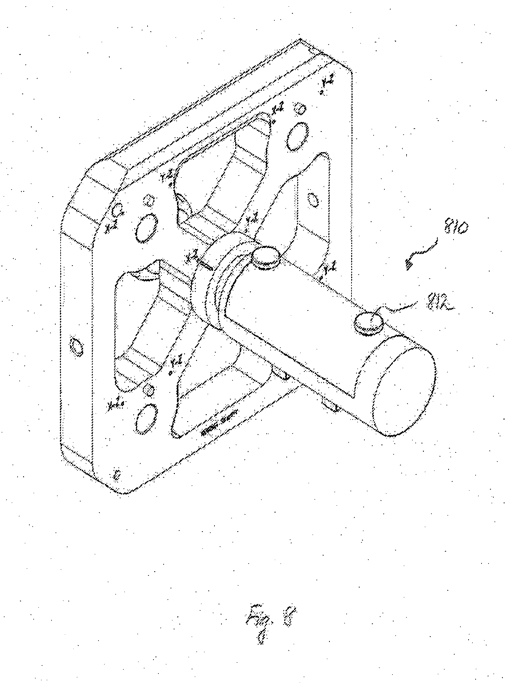

In Fig. 8, a calibration tool 810 comprising reference objects 812 is shown. In this embodiment, the reference objects 812 are, for example, balls, cylinder shaped elements, or marks provided on the inner or outer surface of the cylinder shaped calibration tool 810 or balls provided inside the cylinder shaped calibration tool 810. The reference objects 812 have predetermined positions in the calibration tool 812 and thus have predetermined positions in the stereotactic fixation unit coordinate system when the calibration tool 810 is attached to the fixation arrangement 28.

Returning now to Fig. 2, a processing unit 120 is connectable to the imaging system 50 such two-way communications is allowed, for example, wirelessly using, for example, Bluetooth or WLAN. Thereby, the processing unit 120 may, for example, obtain image information from the imaging system 50 and send instructions to imaging system 50 to initiate an image scanning procedure. The processing unit 120 is inter alia configured to obtain a volumetric reconstruction of the calibration tool and/or a set of images of an area including a calibration tool, e.g. any one of the calibration tools 110, 610, 710, or 810 shown in Fig. 3, 6 - 8, captured by imaging system 50 from the imaging system 50. In the following, reference will be made to the calibration tool 110 shown in Fig. 3.

Further, a position of the calibration tool 110 is determined in a coordinate system related to the imaging system 50 using the volumetric reconstruction of the calibration tool and/or the obtained set of images. The CBCT reconstruction is made with relation to the rotation axis of the imaging system, i.e. the c-axis in Fig. 1. However, the coordinate system of the stereotactic fixation unit (as defined by the axes x, y, and z) in which the calibration tool 110 is positioned is not aligned with the coordinate system of the imaging system (defined by the axes a, b, and c) due to, for example, manufacturing tolerances. Consequently, there will be a position error between the position of the volumetric reconstruction of the calibration tool in the imaging system coordinate system (a, b, c) and the actual position of the calibration tool 110 in the stereotactic fixation unit coordinate system (x, y, and z).

Thus, the processing unit 120 determines the position, a_{cal.tool}, b_{cal.tool}, and c_{cal.tool}, of the calibration tool 110 in the coordinate system of the imaging system 50 or rather the positions of the reference objects, i.e. a set of coordinates is obtained where each reference object is associated with three coordinates. Preferably, the coordinates of each reference object 114 are determined resulting in an array of position coordinates.

Furthermore, the processing unit 120 is configured to calculate a position difference between the determined position of the calibration tool in the coordinate system related to the imaging system, a_{cal.tool}, b_{cal.tool}, and c_{cal.tool}, and a position of the calibration tool in the stereotactic fixation unit coordinate system, x_{cal.tool}, y_{cal.tool}, and z_{cal.tool}, to determine a relationship between the coordinate system related to the imaging system and the position of the calibration tool in the stereotactic fixation unit coordinate system. Preferably, the position difference between the known positions of the reference marks in the stereotactic fixation unit coordinate system and the determined positions of the calibration tool in the coordinate system related to the imaging system are determined.

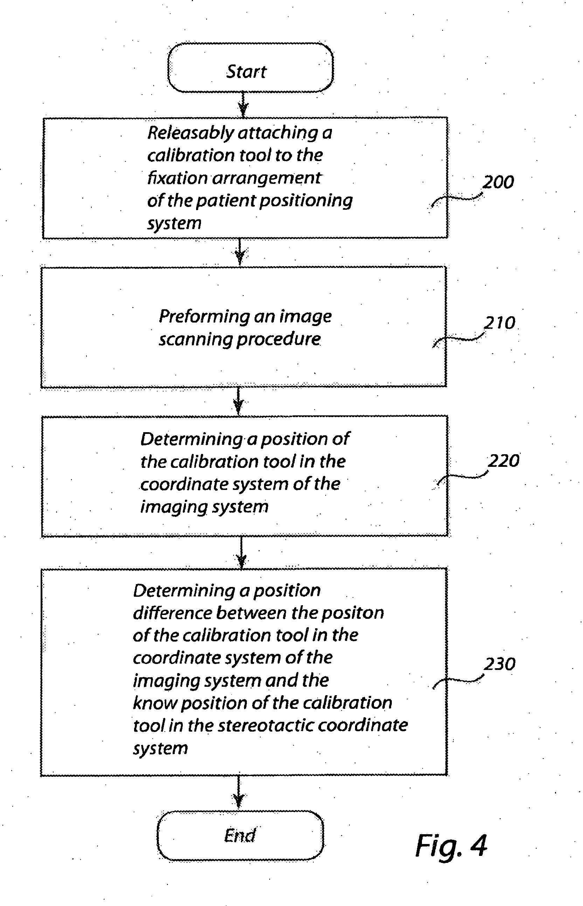

With reference now to Fig.4, the general steps of a method according to the present invention for calibrating an imaging system 50 for capturing images of a patient in connection with treatment planning or treatment in a radiation therapy system will be described. The method is preferably performed in a system as described in Fig. 2.

A first step may be to perform a calibration of image quality parameters of the imaging system 50, which preferably is a CBCT system, including determining a rotational axis of the imaging system 50. Alternatively, if a calibration has been performed earlier, the imaging system 50 may not need a calibration and calibration data can be stored in a calibration file.

At step 200, a calibration tool, e.g. any one of the calibration tools 110, 610, 710, or 810 shown in Fig. 3, 6 - 8, is releasably attached to the fixation arrangement 28, wherein the calibration tool 110 is positioned in the stereotactic fixation unit coordinate system (x, y, z) related to the patient positioning system 20. In the following, the method will be described with reference to the calibration tool 110 shown in Fig. 3.

At step 210, an image scanning procedure is performed with the imaging system 50, wherein a set of images are captured of an area including the calibration tool 110.

Thereafter, at step 220, a position of the calibration tool 110 is determined in the coordinate system (a, b, c) related to the imaging system (50) using the set of images. As has been described above, preferably the positions of the reference objects 112 are determined.

At step 230, a position difference between the determined position of the calibration tool 110 in the coordinate system (a, b, c) related to the imaging system 50 and the known position of the calibration tool 110 in the stereotactic fixation unit coordinate system (x, y, z). Preferably, position differences between the determined positions of the reference objects in the coordinate system (a, b, c) related to the imaging system 50 and the known positions of the reference objects in the stereotactic fixation unit coordinate system (x, y, z) are determined. Thereby, a relationship between the position of the calibration tool 110 in the coordinate system related to the imaging system 50 and the position of the calibration tool 110 in the stereotactic fixation unit coordinate system can be determined.

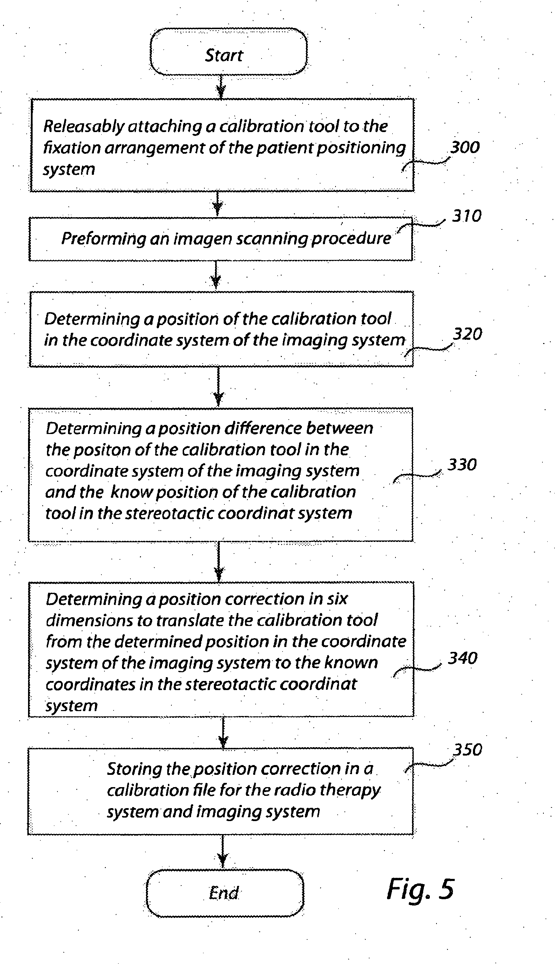

With reference now to Fig.5, the steps of an embodiment of the method according to the present invention for calibrating an imaging system 50 for capturing images of a patient in connection with treatment planning or treatment in a radiation therapy system will be described. The method is preferably performed in a system as described in Fig. 2.

A first step may be to perform a calibration of image quality parameters of the imaging system 50 including determining a rotational axis of the imaging system 50. Alternatively, if a calibration has been performed earlier, the imaging system 50 may not need a calibration and calibration data can be stored in a calibration file.

At step 300, a calibration tool, e.g. any one of the calibration tools 110, 610, 710, or 810 shown in Fig. 3, 6 - 8, is releasably attached to the fixation arrangement 28, wherein the calibration tool 110 is positioned in the stereotactic fixation unit coordinate system (x, y, z) related to the patient positioning system 20. In the following, the method will be described with reference to the calibration tool 110 shown in Fig. 3.

At step 310, an image scanning procedure is performed with the imaging system 50, wherein a set of images are captured of an area including the calibration tool 110. This includes determining a volumetric reconstruction of the area includig the calibration tool (110).

Thereafter, at step 320, a position of the calibration tool 110 is determined in the coordinate system (a, b, c) related to the imaging system (50) using the set of images. As has been described above, preferably the positions of the reference objects 112 are determined.

At step 330, a position difference between the determined position of the calibration tool 110 in the coordinate system (a, b, c) related to the imaging system 50 and the known position of the calibration tool 110 in the stereotactic fixation unit coordinate system (x, y, z). Preferably, position differences between the determined positions of the reference objects in the coordinate system (a, b, c) related to the imaging system 50 and the known positions of the reference objects in the stereotactic fixation unit coordinate system (x, y, z) are determined. Thereby, a relationship between the position of the calibration tool 110 in the coordinate system related to the imaging system 50 and the position of the calibration tool 110 in the stereotactic fixation unit coordinate system can be determined.

In step 340, a position correction is determined based on the calculated position difference. The position correction may correspond to a movement correction for imaging system 50 relative the positioning system (20) required to transform a position in the coordinate system of the imaging system to the corresponding position in the stereotactic fixation unit coordinate system. That is, a movement correction for the calibration tool 110 required to translate the calibration tool 110 from the position in the coordinate system of the imaging system 50 to the known position in the stereotactic fixation unit coordinate system is determined. The position correction comprises determining the movement correction as three rotations with respect to the respective axes x, y and z of the stereotactic fixation unit coordinate system and three translations along the respective axes of the stereotactic fixation unit coordinate system. The position correction can be stored in a calibration file for the imagning system 50 in step 350. For a reconstruction made of an object, for example, a part of a patient, each reconstructed voxel can be transformed from the coordinate system (a, b, c) of the imaging system 50 of the stereotactic coordinate system (x, y, z) using the calibration file.

Even though the present invention has been described above using exemplifying embodiments thereof, alterations, modifications, and combinations thereof, as understood by those skilled in the art, may be made without departing from the scope of the invention as defined in the accompanying claims.

## Claims

1. A method for calibrating an imaging system (50) for capturing images of a patient in connection with treatment planning or treatment in a radiation therapy system (1), which radiation therapy system comprises a radiation therapy unit (10) having a fixed radiation focus point, a positioning system (20) for positioning a treatment volume in a patient in relation to said fixed focus point in the radiation therapy unit (10) along three substantially orthogonal motional axes, wherein said positioning system includes a fixation arrangement (28) for releasably and firmly engaging a stereotactic fixation unit for immobilizing a treatment volume in a patient in relation to the positioning system (20), said method comprising the steps of:
releasably attaching a calibration tool (110) to said fixation arrangement (28), wherein said calibration tool (110) is positioned in a stereotactic coordinate system related to said patient positioning system (20), wherein the calibration tool (110) comprises reference objects (112) arranged or shaped such that they can be identified in a reconstructed volume;
performing an image scanning procedure with said imaging system (50), wherein a set of images are captured of an area including said calibration tool (110);
performing a volumetric reconstruction of said area including the calibration tool (110) using the captured images;
determining a position of said calibration tool (110) in an imaging system coordinate system using said volumetric reconstruction;
calculating a position difference between said determined position of said calibration tool (110) in said imaging system coordinate system and a position of said calibration tool (110) in said stereotactic coordinate system, thereby determining a relationship between said imaging system coordinate system and said position of said calibration tool (110) in said stereotactic coordinate system.

2. The method according to claim 1, further comprising the step of:
performing a calibration of the imaging system (50) including determining a rotational axis of said imaging system (50).

3. The method according to claim 1 or 2, further comprising determining a position correction based on said calculated position difference, said position correction corresponding to a movement correction for said imaging system (50) relative said positioning system (20) required to transform a position in said imaging system coordinate system to the corresponding position in said stereotactic coordinate system.

4. The method according to claim 3, wherein said step of determining a position correction comprises determining the movement correction as three rotations with respect to the respective axes of the stereotactic coordinate system and three translations along the respective axes of the stereotactic coordinate system.

5. The method according to claim 1 - 4, wherein said step of determining a position of said calibration tool (110) in a imaging system coordinate system using said set of images comprises the steps of:
identifying predetermined reference objects of said calibration tool in said volumetric reconstruction, wherein said reference objects have known coordinates in said stereotactic coordinate system; and
determining the coordinates for each identified reference object in said volumetric reconstruction with respect to said imaging system coordinate system

6. The method according to claim 5, wherein the step of calculating a position difference comprises the step of:
comparing coordinates for each identified reference object in said imaging system coordinate system and the corresponding coordinates of said reference objects in said stereotactic coordinate system; and
calculating said position difference between said determined position of said calibration tool in said imaging system coordinate system and a position of said calibration tool in said stereotactic coordinate system based on said comparison.

7. A system for calibrating an imaging system (50) for capturing images of a patient in connection with treatment planning or treatment in a radiation therapy system (1), which radiation therapy system comprises a radiation therapy unit (10) having a fixed radiation focus point, a positioning system (20) for positioning a treatment volume in a patient in relation to said fixed focus point in the radiation therapy unit (10) along three substantially orthogonal motional axes, wherein said positioning system includes fixation arrangements (28) for releasably and firmly engaging a stereotactic fixation unit for immobilizing a treatment volume in a patient in relation to the positioning system (20), wherein the system for calibration comprises:
a calibration tool (110) arranged to be releasably attached to said fixation arrangement (28), wherein said calibration tool (110) is positioned in a stereotactic coordinate system related to said positioning system (20), wherein the calibration tool (110) comprises reference objects (112) arranged or shaped such that they can be identified in a reconstructed volume; **characterised by**:
a processing unit (120) configured to:
obtain a set of images of an area including said calibration tool (110) captured by said imaging device from said imaging system (50);
perform a volumetric reconstruction of said area including the calibration tool (110) using the captured images;
determine a position of said calibration tool (110) in a imaging system coordinate system using the volumetric reconstruction;
calculate a position difference between said determined position of said calibration tool (110) in said imaging system coordinate system and a position of said calibration tool (110) in said stereotactic coordinate system to determine a relationship between said imaging system coordinate system and said position of said calibration tool (110) in said stereotactic coordinate system.

8. The system according to claim 7, wherein said calibration tool comprises:
attachment means (118) for enabling releasable attachment to said fixation arrangement (28); and
reference objects (112) having a shape enabling a position determination in six dimensions.

9. The system according to claim 8, wherein calibration tool comprises at least three reference objects each including a rod (116) attached to a plate (119), said plate comprising said attachment means (118), and a ball (115) attached to the rod.

10. The system according to claim 7 - 9, wherein said processing unit (120) is further configured to determine a position correction based on said calculated position difference, said position correction corresponding to a movement correction for said imaging system (50) relative said positioning system (20) required to transform a position in said imaging system coordinate system to the corresponding position in said stereotactic coordinate system.

11. The system according to claim 10, wherein said processing unit (120) is further configured to determine the movement correction as three rotations with respect to the respective axes of the stereotactic coordinate system and three translations along the respective axes of the stereotactic coordinate system.

12. The system according to claim 7 - 11, wherein said processing unit (120) is further configured to:
identify predetermined reference objects of said calibration tool in said volumetric reconstruction, wherein said reference objects have known coordinates in said stereotactic coordinate system; and
determine the coordinates for each identified reference object in said volumetric reconstruction with respect to said imaging system coordinate system.

13. The system according to claim 12, wherein said processing unit (120) is further configured to:
compare coordinates for each identified reference object in said imaging system coordinate system and the corresponding coordinates of said reference objects in said stereotactic coordinate system; and
calculate said position difference between said determined position of said calibration tool in said imaging system coordinate system and a position of said calibration tool in said stereotactic coordinate system based on said comparison.

## Patentansprüche

1. Verfahren zum Kalibrieren eines Imagingsystems (50) zum Aufnehmen von Bildern eines Patienten in Verbindung mit Behandlungsplanung oder Behandlung in einem Strahlungstherapiesystem (1), wobei das Strahlungstherapiesystem eine Strahlungstherapieeinheit (10) mit einem festen Strahlungsbrennpunkt und ein Positionierungssystem (20) zum Positionieren eines Behandlungsvolumens in einem Patienten relativ zu dem festen Brennpunkt in der Strahlungstherapieeinheit (10) entlang dreier im Wesentlichen orthogonaler Bewegungsachsen umfasst, wobei das Positionierungssystem eine Befestigungseinrichtung (28) zum freigebbaren und festen Eingreifen einer stereotaktischen Befestigungseinheit zum Immobilisieren eines Behandlungsvolumens in einem Patienten relativ zu dem Positionierungssystem (20) enthält, wobei das Verfahren die folgenden Schritte umfasst:
freigebbares Anbringen eines Kalibrationswerkzeugs (110) an der Befestigungseinrichtung (28), wobei das Kalibrationswerkzeug (110) in einem stereotaktischen Koordinatensystem relativ zu dem Patientenpositionierungssystem (20) positioniert ist,
wobei das Kalibrationswerkzeug (110) Referenzobjekte (112) umfasst, die so angeordnet oder geformt sind, dass sie in einem rekonstruierten Volumen identifiziert werden können;
Durchführen einer Bildscanprozedur mit dem Imagingssystem (50), wobei eine Menge von Bildern eines das Kalibrationswerkzeug (110) enthaltenden Gebiets aufgenommen wird;
Durchführen einer volumetrischen Rekonstruktion des das Kalibrationswerkzeug (110) enthaltenden Gebiets unter Verwendung der aufgenommenen Bilder;
Bestimmen einer Position des Kalibrationswerkzeugs (110) in einem Imagingsystem-Koordinatensystem unter Verwendung der volumetrischen Rekonstruktion;
Berechnen einer Positionsdifferenz zwischen der bestimmten Position des Kalibrationswerkzeugs (110) in dem Imagingsystem-Koordinatensystem und einer Position des Kalibrationswerkzeugs (110) in dem stereotaktischen Koordinatensystem, wodurch eine Beziehung zwischen dem Imagingsystem-Koordinatensystem und den entsprechenden Koordinaten des Referenzobjekts in dem stereotaktischen Koordinatensystem bestimmt wird; und
Berechnen der Positionsdifferenz zwischen der bestimmten Position des Kalibrationswerkzeugs in dem Imagingsystem-Koordinatensystem und der Position des Kalibrationswerkzeugs (110) in dem stereotaktischen Koordinatensystem.

2. Verfahren nach Anspruch 1, ferner umfassend den Schritt des Durchführens einer Kalibration des Imagingsystems (50) einschließlich des Bestimmens einer Rotationsachse des Imagingsystems (50).

3. Verfahren nach Anspruch 1 oder 2, ferner umfassend Bestimmen einer Positionskorrektur auf der Basis der berechneten Positionsdifferenz, wobei die Positionskorrektur einer Bewegungskorrektur für das Imagingsystem (50) relativ zu dem Positionierungssystem (20) entspricht, die erforderlich ist, um eine Position in dem Imagingsystem-Koordinatensystem in eine entsprechende Position in dem stereotaktischen Koordinatensystem umzuwandeln.

4. Verfahren nach Anspruch 3, wobei der Schritt des Bestimmens einer Positionskorrektur Bestimmen der Bewegungskorrektur als drei Drehungen relativ zu den entsprechenden Achsen des stereotaktischen Koordinatensystems und drei Verschiebungen entlang den entsprechenden Achsen des stereotaktischen Koordinatensystems umfasst.

5. Verfahren nach Ansprüchen 1 bis 4, wobei der Schritt des Bestimmens einer Position des Kalibrationswerkzeugs (110) in einem Imagingsystem-Koordinatensystem unter Verwendung der Menge von Bildern die folgenden Schritte umfasst:
Identifizieren vorbestimmter Referenzobjekte des Kalibrationswerkzeugs in der volumetrischen Rekonstruktion, wobei die Referenzobjekte bekannte Koordinaten in dem stereotaktischen Koordinatensystem aufweisen; und
Bestimmen der Koordinaten für jedes identifizierte Referenzobjekt in der volumetrischen Rekonstruktion relativ zu dem Imagingsystem-Koordinatensystem.

6. Verfahren nach Anspruch 5, wobei der Schritt des Berechnens einer Positionsdifferenz die folgenden Schritte umfasst:
Vergleichen von Koordinaten für jedes identifizierte Referenzobjekt in dem Imagingsystem-Koordinatensystem und der entsprechenden Koordinaten der Referenzobjekte in dem stereotaktischen Koordinatensystem; und
Berechnen der Positionsdifferenz zwischen der bestimmten Position des Kalibrationswerkzeugs in dem Imagingsystem-Koordinatensystem und einer Position des Kalibrationswerkzeugs in dem stereotaktischen Koordinatensystem basierend auf dem Vergleich.

7. System zum Kalibrieren eines Imagingsystems (50) zum Aufnehmen von Bildern eines Patienten in Verbindung mit Behandlungsplanung oder Behandlung in einem Strahlungstherapiesystem (1), wobei das Strahlungstherapiesystem eine Strahlungstherapieeinheit (10) mit einem festen Strahlungsbrennpunkt und ein Positionierungssystem (20) zum Positionieren eines Behandlungsvolumens in einem Patienten relativ zu dem festen Brennpunkt in der Strahlungstherapieeinheit (10) entlang dreier im Wesentlichen orthogonaler Bewegungsachsen umfasst, wobei das Positionierungssystem eine Befestigungseinrichtung (28) zum freigebbaren und festen Eingreifen einer stereotaktischen Befestigungseinheit zum Immobilisieren eines Behandlungsvolumens in einem Patienten relativ zu dem Positionierungssystem (20) enthält, wobei das System zur Kalibration Folgendes umfasst:
ein Kalibrationswerkzeug (110), angeordnet, freigebbar an der Befestigungseinrichtung (28) angebracht zu werden, wobei das Kalibrationswerkzeug (110) in einem stereotaktischen Koordinatensystem relativ zu dem Patientenpositionierungssystem (20) positioniert ist,
wobei das Kalibrationswerkzeug (110) Referenzobjekte (112) umfasst, die so angeordnet oder geformt sind, dass sie in einem rekonstruierten Volumen identifiziert werden können; gekennzeichnet durch:
eine Verarbeitungseinheit (120), ausgelegt,
eine Menge von Bildern eines das Kalibrationswerkzeug (110) enthaltenden Gebiets zu erhalten, die von der Imagingeinrichtung des Imagingsystems (50) aufgenommen wurde;
eine volumetrische Rekonstruktion des das Kalibrationswerkzeug (110) enthaltenden Gebiets unter Verwendung der aufgenommenen Bilder durchzuführen;
eine Position des Kalibrationswerkzeugs (110) in einem Imagingsystem-Koordinatensystem unter Verwendung der volumetrischen Rekonstruktion zu bestimmen;
eine Positionsdifferenz zwischen der bestimmten Position des Kalibrationswerkzeugs (110) in dem Imagingsystem-Koordinatensystem und einer Position des Kalibrationswerkzeugs (110) in dem stereotaktischen Koordinatensystem zu berechnen, um eine Beziehung zwischen dem Imagingsystem-Koordinatensystem und der Position des Kalibrationswerkzeugs (110) in dem stereotaktischen Koordinatensystem zu bestimmen.

8. System nach Anspruch 7, wobei das Kalibrationswerkzeug umfasst:
Befestigungsmittel (118) zum Ermöglichen freigebbarer Anbringung an der Befestigungseinrichtung (28); und
Referenzobjekte (112) mit einer Form, die Positionsbestimmung in sechs Dimensionen ermöglicht.

9. System nach Anspruch 8, wobei das Kalibrationswerkzeug mindestens drei Referenzobjekte umfasst, wobei jedes einen an einer Platte (119) angebrachten Stab (116) beinhaltet, wobei die Platte die Befestigungsmittel (118) und eine an dem Stab angebrachte Kugel (115) umfasst.

10. System nach Ansprüchen 7 - 9, wobei die Verarbeitungseinheit (120) ferner ausgelegt ist, eine Positionskorrektur auf der Basis der berechneten Positionsdifferenz zu bestimmen, wobei die Positionskorrektur einer Bewegungskorrektur für das Imagingsystem (50) relativ zu dem Positionierungssystem (20) entspricht, die erforderlich ist, um eine Position in dem Imagingsystem-Koordinatensystem in eine entsprechende Position in dem stereotaktischen Koordinatensystem umzuwandeln.

11. System nach Anspruch 10, wobei die Verarbeitungseinheit (120) ferner ausgelegt ist, eine Bewegungskorrektur als drei Drehungen relativ zu den entsprechenden Achsen des stereotaktischen Koordinatensystems und drei Verschiebungen entlang den entsprechenden Achsen des stereotaktischen Koordinatensystems zu bestimmen.

12. System nach Ansprüchen 7 - 11, wobei die Verarbeitungseinheit (120) ferner ausgelegt ist, vorbestimmte Referenzobjekte des Kalibrationswerkzeugs in der volumetrischen Rekonstruktion zu identifizieren, wobei die Referenzobjekte bekannte Koordinaten in dem stereotaktischen Koordinatensystem aufweisen; und
die Koordinaten für jedes identifizierte Referenzobjekt in der volumetrischen Rekonstruktion relativ zu dem Imagingsystem-Koordinatensystem zu bestimmen.

13. System nach Anspruch 12, wobei die Verarbeitungseinheit (120) ferner ausgelegt ist, Koordinaten für jedes identifizierte Referenzobjekt in dem Imagingsystem-Koordinatensystem und die entsprechenden Koordinaten der Referenzobjekte in dem stereotaktischen Koordinatensystem zu vergleichen; und die Positionsdifferenz zwischen der bestimmten Position des Kalibrationswerkzeugs in dem Imagingsystem-Koordinatensystem und einer Position des Kalibrationswerkzeugs in dem stereotaktischen Koordinatensystem basierend auf dem Vergleich zu berechnen.

## Revendications

1. Procédé d'étalonnage d'un système de formation d'image (50) destiné à acquérir des images d'un patient en association avec une planification de traitement ou un traitement dans un système de radiothérapie (1), lequel système de radiothérapie comprend une unité de radiothérapie (10) comportant un point focal de rayonnement fixe, un système de positionnement (20) destiné à positionner un volume de traitement dans un patient par rapport audit point focal fixe dans l'unité de radiothérapie (10) le long de trois axes de mouvement sensiblement orthogonaux, dans lequel ledit système de positionnement comprend un moyen de fixation (28) destiné à venir en prise de manière amovible et ferme avec une unité de fixation stéréotaxique destinée à immobiliser un volume de traitement dans un patient par rapport au système de positionnement (20), ledit procédé comprenant les étapes consistant à :
fixer de manière amovible un outil d'étalonnage (110) audit moyen de fixation (28), dans lequel ledit outil d'étalonnage (110) est positionné dans un référentiel stéréotaxique lié audit système de positionnement de patient (20), dans lequel l'outil d'étalonnage (110) comprend des objets de référence (112) disposés ou
configurés de manière à ce qu'ils puissent être identifiés dans un volume reconstruit ;
effectuer un processus de balayage d'image avec ledit système de formation d'image (50), dans lequel un ensemble d'images d'une zone comprenant ledit outil d'étalonnage (110) sont acquises ;
effectuer une reconstruction volumétrique de ladite zone comprenant l'outil d'étalonnage (110) en utilisant les images acquises ;
déterminer une position dudit outil d'étalonnage (110) dans un référentiel du système de formation d'image en utilisant ladite reconstruction volumétrique ;
calculer une différence de position entre ladite position déterminée dudit outil d'étalonnage (110) dans ledit référentiel du système de formation d'image et
une position dudit outil d'étalonnage (110) dans ledit référentiel stéréotaxique, pour déterminer ainsi une relation entre ledit référentiel du système de formation d'image et ladite position dudit outil d'étalonnage (110) dans ledit référentiel stéréotaxique.

2. Procédé selon la revendication 1, comprenant en outre l'étape consistant à :
effectuer un étalonnage du système de formation d'image (50), cela consistant à déterminer un axe de rotation dudit système de formation d'image (50).

3. Procédé selon la revendication 1 ou 2, consistant en outre à déterminer une correction de position sur la base de ladite différence de position calculée, la correction de position correspondant à une correction de mouvement pour ledit système de formation d'image (50) par rapport audit système de positionnement (20), exigée pour transformer une position dans ledit référentiel du système de formation d'image en la position correspondante dans ledit référentiel stéréotaxique.

4. Procédé selon la revendication 3, dans lequel ladite étape de détermination d'une correction de position consiste à déterminer la correction du mouvement sous la forme de trois rotations par rapport aux axes respectifs du référentiel stéréotaxique et de trois translations le long des axes respectifs du référentiel stéréotaxique.

5. Procédé selon les revendications 1-4, dans lequel ladite étape de détermination d'une position dudit outil d'étalonnage (110) dans un référentiel du système de formation d'image en utilisant ledit ensemble d'images comprend les étapes consistant à :
identifier des objets de référence prédéterminés dudit outil d'étalonnage dans ladite reconstruction volumétrique, dans lequel lesdits objets de référence ont des coordonnées connues dans ledit référentiel stéréotaxique ; et
déterminer les coordonnées correspondant à chaque objet de référence identifié dans ladite reconstruction volumétrique par rapport audit référentiel du système de formation d'image.

6. Procédé selon la revendication 5, dans lequel l'étape de calcul d'une différence de position comprend les étapes consistant à :
comparer des coordonnées pour chaque objet de référence identifié dans ledit référentiel du système de formation d'image et les coordonnées correspondantes desdits objets de référence dans ledit référentiel stéréotaxique ; et
calculer la différence de position entre ladite position déterminée dudit outil d'étalonnage dans ledit référentiel du système de formation d'image et une position dudit outil d'étalonnage dans ledit référentiel stéréotaxique sur la base de ladite comparaison.

7. Système d'étalonnage d'un système de formation d'image (50) destiné à acquérir des images d'un patient en association avec une planification de traitement ou un traitement dans un système de radiothérapie (1), lequel système de radiothérapie comprend une unité de radiothérapie (10) comportant un point focal de rayonnement fixe, un système de positionnement (20) destiné à positionner un volume de traitement dans un patient par rapport audit point focal fixe dans l'unité de radiothérapie (10) le long de trois axes de mouvement sensiblement orthogonaux, dans lequel ledit système de positionnement comprend des moyens de fixation (28) destinés à venir en prise de manière amovible et ferme avec une unité de fixation stéréotaxique destinée à immobiliser un volume de traitement dans un patient par rapport au système de positionnement (20), dans lequel le système d'étalonnage comprend :
un outil d'étalonnage (110) disposé afin d'être fixé de manière amovible audit moyen de fixation (28), dans lequel ledit outil d'étalonnage (110) est positionné dans un référentiel stéréotaxique par rapport audit système de positionnement (20), dans lequel l'outil d'étalonnage (110) comprend des objets de référence (112) agencés ou configurés de manière à ce qu'ils puissent être identifiés dans un volume reconstruit ;
**caractérisé par** :
une unité de traitement (120) configurée pour :
obtenir un ensemble d'images d'une zone comprenant ledit outil d'étalonnage (110) acquis par ledit dispositif de formation d'image en provenance dudit système de formation d'image (50) ;
effectuer une reconstruction volumétrique de ladite zone comprenant l'outil d'étalonnage (110) en utilisant les images acquises ;
déterminer une position dudit outil d'étalonnage (110) dans un référentiel du système de formation d'image en utilisant la reconstruction volumétrique ;
calculer une différence de position entre ladite position déterminée dudit outil d'étalonnage (110) dans ledit référentiel du système de formation d'image et
une position dudit outil d'étalonnage (110) dans ledit référentiel stéréotaxique, pour déterminer une relation entre ledit référentiel du système de formation d'image et ladite position dudit outil d'étalonnage (110) dans ledit référentiel stéréotaxique.

8. Système selon la revendication 7, dans lequel ledit outil d'étalonnage comprend :
un moyen de fixation (118) pour permettre une fixation amovible audit moyen de fixation (28) ; et
des objets de référence (112) ayant une forme permettant une détermination de position en six dimensions.

9. Système selon la revendication 8, dans lequel l'outil d'étalonnage comprend au moins trois objets de référence comportant chacun une tige (116) fixée à une plaque (119), ladite plaque comprenant ledit moyen de fixation (118) et une bille (115) fixée à la tige.

10. Système selon les revendications 7 - 9, dans lequel ladite unité de traitement (120) est en outre configurée pour déterminer une correction de position sur la base de ladite différence de position calculée, ladite correction de position correspondant à une correction du mouvement pour ledit système de formation d'image (50) par rapport audit système de positionnement (20) exigée pour transformer une position dans ledit référentiel du système de formation d'image, en la position correspondante dans ledit référentiel stéréotaxique.

11. Système selon la revendication 10, dans lequel ladite unité de traitement (120) est en outre configurée pour déterminer la correction de mouvement sous la forme de trois rotations par rapport aux axes respectifs du référentiel stéréotaxique et trois translations le long des axes respectifs du référentiel stéréotaxique.

12. Système selon les revendications 7 - 11, dans lequel ladite unité de traitement (120) est en outre configurée pour :
identifier des objets de référence prédéterminés dudit outil d'étalonnage lors de ladite reconstruction volumétrique, dans lequel lesdits objets de référence ont des coordonnées connues dans ledit référentiel stéréotaxique ; et
déterminer les coordonnées pour chaque objet de référence identifié dans ladite reconstruction volumétrique par rapport audit référentiel du système de formation d'image.

13. Système selon la revendication 12, dans lequel ladite unité de traitement (120) est en outre configurée pour :
comparer des coordonnées, pour chaque objet de référence identifié, dans ledit référentiel du système de formation d'image et les coordonnées correspondantes desdits objets de référence dans ledit référentiel stéréotaxique ; et
calculer ladite différence de position entre ladite position déterminée dudit outil d'étalonnage dans ledit référentiel du système de formation d'image et une position dudit outil d'étalonnage dans ledit référentiel stéréotaxique sur la base de ladite comparaison.
